# EUROPEAN PATENT APPLICATION

(11) **EP 2 016 927 A2**
(43) Date of publication of application: **21.01.2009**
(21) Application number: 08011989.4
(22) Date of filing: 03.07.2008
(51) Int. Cl.: A61H 23/04

(54) **Garment for thermotherapy, cryotherapy and pressotherapy treatments**

(30) Priority: 03.07.2007 ES 200701430 U
(71) Applicant: Suministros Tecnicos Para La Belleza, S.L., 08907 L'Hospitalet de Llobregat (ES)
(72) Inventor: Moreno, Manuel Ayllon, 08907 L'Hospitalet de Llobregat (ES)
(74) Representative: Torras Toll, Jorge

(57) **Abstract**

Garment for thermotherapy, cryotherapy and pressotherapy treatments, of the type used in health centres, spas and suchlike, essentially characterised in that it consists of the connection of two functional elements positioned concentrically one around the other, and both together around the user, the first element being a pressotherapy applicator (1) that is situated on the outside of the garment, and the other element being the thermotherapy and cryotherapy (2) applicator that is situated on the inside of the garment.

## Description

### TECHNICAL FIELD

The object of the present invention, as declared in the statement of this specification, relates to a garment for thermotherapy, cryotherapy and pressotherapy treatments, of the type used at health centres, spas and suchlike, which is particularly indicated for use in medical and/or beauty applications.

### BACKGROUND OF THE INVENTION

Traditionally, pressotherapy is based on sequentially applying massage by air pressure or by pressure from any other fluid to different parts of the body; there are special types of garments on the market for this, such as trousers or boots (for leg, groin and/or buttocks treatments), abdominal garments, garments for arms, and even jackets (which enables the combined treatment of back, abdomen and arms). The garment consists of a set of separate sealed chambers that can overlap each other or not, positioned in a suitable way so that when activated (i.e. inflating and/or deflating them separately) according to a certain sequence, they result in the desired medical or beauty treatment. The effect produced by said treatment is the generation of a massage that is capable of moving body fluids, acting deeply at muscular level and achieving a lymphatic drainage. The pressures are controlled by a compressor governed by a microprocessor, which sequentially provides different levels of pressure to the different chambers according to a pre-established programme for each type of massage.

Cryotherapy is a type of thermal treatment that consists of generating and applying cold to the body; its purpose is to achieve different therapeutic effects. There is a range of different cryotherapy equipment on the market, both for treating very localised small areas of the body and for treating large areas; in the latter case, the purpose is to reduce the body temperature in order to treat excess weight, combat analgesia and other conditions, etc. The generation of cold is currently based on either the use of a compressor that propels a cold fluid through a coil that is in contact with the body surface to be treated, or "Peltier" effect cells (cooling by electrical current flow) that are in contact with said surface, or a combination of both techniques. Said cold is normally applied to the body by using special garments provided with the aforementioned cooling systems.

A second type of thermal treatment is thermotherapy, the purpose of which is to generate and apply heat to certain parts of the body to increase their temperature in order to achieve certain therapeutic effects such as eliminating toxins by perspiration, treatment for excess weight, etc. Thermotherapy application techniques are similar to the cryotherapy technique, i,e. either the use of a compressor that propels a hot fluid along a coil, or heating using a flow of electrical current, or with other accessories such as electric blankets and sauna bags. The heat is normally applied to the body using special garments provided with said heating systems.

However, all these devices or garments have a very specific disadvantage: if all three treatments are to be applied, this can only be done separately and sequentially, which means that the user has to have the three separate devices and use first one, then the second and lastly the third, with the drawbacks of the inconvenience and time-wasting that this involves, in addition to the economic cost and the space taken up by having the three separate devices. Furthermore, the combination of several simultaneously-applied therapies has a synergic effect, achieving better results than by applying the same therapies individually.

### DESCRIPTION OF THE INVENTION

In order to overcome these disadvantages, the novel garment for thermotherapy, cryotherapy and pressotherapy treatments, and application of thermal contrasts, which is the object of the present specification.

In general terms, the present invention relates to a garment that includes the necessary elements to apply any of the three treatments, either sequentially or at the same time: pressure, heat, cold, pressure and heat, pressure and cold, or also increasing thermal contrasts: heat/cold and cold/heat, or pressure/heat and pressure/cold.

The advantage of the new device is that it is possible to apply the three treatments with a single garment, which means that it is not necessary to have three separate garments and the garment need not be changed when passing from treatment to the next; it is also more economical than the other three garments separately and its installation occupies less space.

In theory, the novel garment takes the form of trousers, although it can also optionally be a jacket, an entire suit, or any combination of the aforementioned garments or others that might be developed.

The novel garment essentially consists of the connection of two fundamental elements placed concentrically one around the other, and both together around the user; one element is the pressotherapy applicator (situated on the outside of the garment) and the other element is the thermotherapy and cryotherapy applicator (situated on the inside of the garment).

The pressotherapy applicator element consists of an impermeable fabric (plastic or suchlike) divided into a set of separate compartments or sealed chambers that are evenly distributed and exteriorly protected by a cover (which may cover one or both faces: inside and outside); each chamber has a connector to which a hose can be connected to enable the independent inflation thereof to apply the desired pressure; all the hoses from the chambers are connected (by an outer multiple connector) to a distributor device and/or a set of electrovalves that regulate the different pressures, making it possible to fill the different chambers sequentially; this device is in turn connected to a compressor. For aesthetic reasons and for the user's comfort, all the hoses from each leg of the trouser garment are gathered and channelled preferably towards the inside of the leg inside an unfolding flap with a closure that is preferably of the velcro type.

The cryotherapy and thermotherapy applicator element, which is attached to the inside of the pressotherapy element (and concentrically thereto) by a zip and/or velcro, consists of a coil of hose positioned between two covers: one inner and one outer; this coil is responsible for carrying the heating and/or cooling fluid to the inside of the leg; the inner cover is made from fabrics that are good temperature conductors in order to achieve a better application of cold or heat to the user; the outside is provided with the necessary devices to heat or cool the fluid; the cryotherapy and thermotherapy element has a tube for connection to said devices, which is separate from that used in the connection of the tubes of the pressotherapy element.

Optionally, it is also possible to use electrical resistances and "Peltier" cells as a heating and/or cooling system, or any other heat and/or cold transfer device that is considered suitable for these therapeutic applications.

The outer pressotherapy applicator element and inner cold and/or heat applicator element are joined together by a zip and/or velcro; thus, the garment as a whole is strong enough to be used comfortably.

The novel garment is made in such a way that each of the two legs (when the garment takes the form of trousers) is vertically open, preferably along the outside thereof, so that it is easy for the user to put on; each of these vertical openings has several zips or velcros (preferably three), so that the legs can be adjusted to the diameter of each user's leg.

### DESCRIPTION OF THE DRAWINGS

In order to illustrate the above, the present description is accompanied by a set of simplified, schematic drawings that show a non-limiting explanatory example of a practical embodiment of the characteristics of the novel invention. This practical example corresponds to a garment according to the invention in the form of trousers.

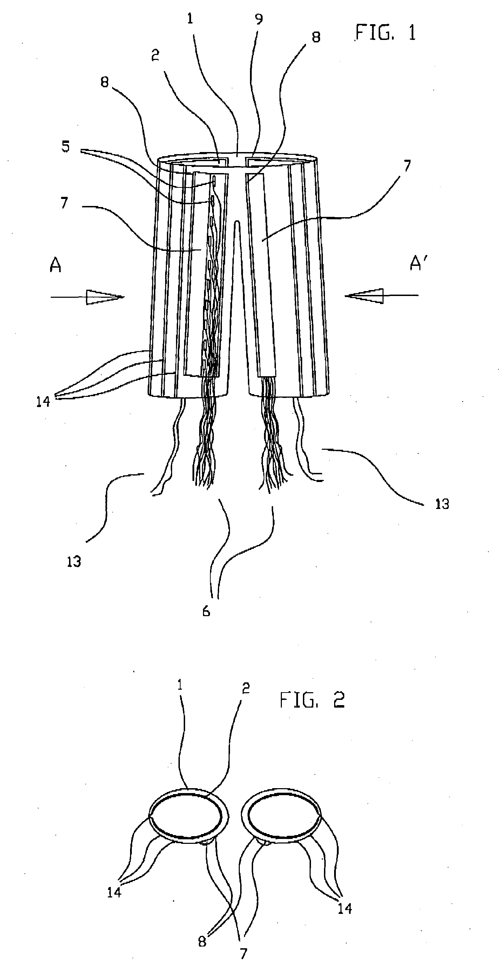

- Figure 1: shows a perspective front view from above of the novel garment when closed; it is possible to observe that the unfolding flap on the right is closed, whereas the one on the left is open and with all the pressotherapy tubes on view.
- Figure 2: shows a perspective view from above of a cross section taken through A-A' of figure 1.

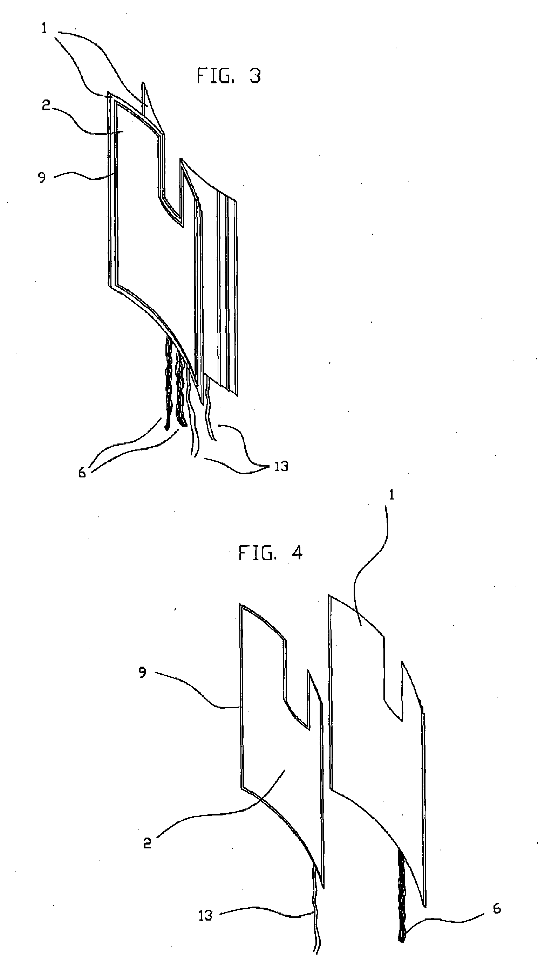

- Figure 3: shows a perspective view of the garment when open on each side.
- Figure 4: shows a perspective view of the two elements that make up one of the two legs, separately in an exploded view: the outer pressotherapy element (on the right) with its set of tubes, and the inner cryo- thermotherapy element (on the left) with its inlet and outlet tube.

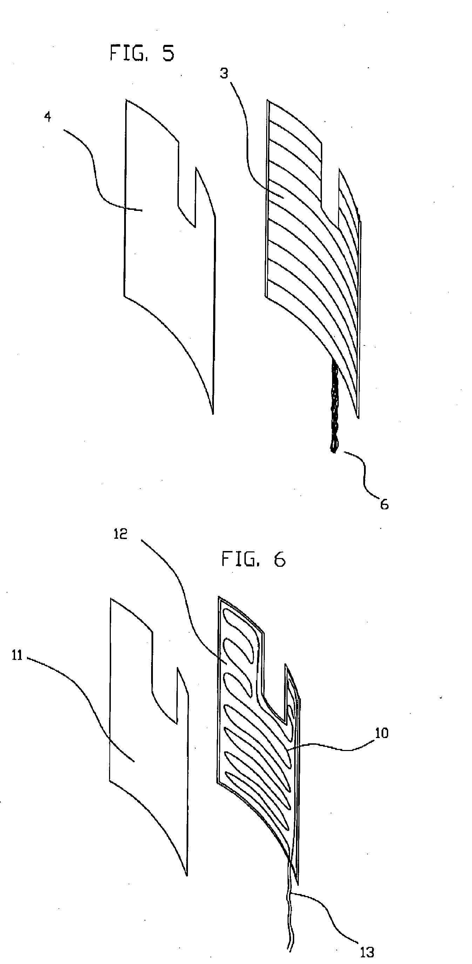

- Figure 5: shows a perspective view of the two components that make up the pressotherapy element, separated in an exploded view: the compartmented sealed fabric (on the right) and its inner cover (on the left).
- Figure 6: shows a perspective view of the two components that make up the cryo-thermotherapy element, separated in an exploded view: the coil (on the right) and its inner cover (on the left).

### DESCRIPTION OF A PRACTICAL EXAMPLE

The figures that accompany the present specification disclose, by way of an example, a practical embodiment of the device that is the object thereof.

The novel trouser-type garment essentially consists of the connection of two fundamental elements positioned concentrically one around the other, and both around the user, one element is the pressotherapy applicator (1), which is situated on the outside of the garment, and the other element is the thermotherapy and cryotherapy applicator (2), which is situated on the inside of the garment.

The pressotherapy applicator element is made from an impermeable fabric (3), such as plastic or suchlike, which is divided into a set of separate compartments or sealed chambers that are evenly distributed, exteriorly protected by a cover (4) that can cover one or both faces: inside and outside; each chamber has a connector (5) to which a hose (6) can be connected to enable the independent inflation thereof to apply the desired pressure; all the hoses (6) from the chambers are connected (by an outer multiple connector) to a distributor device and/or a set of electrovalves that regulate the different pressures, making it possible to fill the different chambers (3) sequentially; this device is in turn connected to a compressor. For aesthetic reasons and for the user's comfort, all the hoses (6) from each leg of the trouser garment are gathered and channelled preferably towards the inside of the leg inside an unfolding flap (7) with a closure (8) that is preferably of the velcro type provided on the front face of the cover (4).

The cryotherapy and thermotherapy applicator element (2), which is attached to the inside of the pressotherapy element (1), and concentrically thereto, by a zip and/or velcro (9), consists of a coil (10) of hose positioned between two covers: one inner (11) and one outer (12); this coil (10) is responsible for carrying the heating and/or cooling fluid to the inside of the leg; the inner cover (11) is made from fabrics that are good temperature conductors in order to achieve a better application of cold or heat to the user; the outside is provided with the necessary devices to heat or cool the fluid; the cryotherapy and thermotherapy element has a tube (13) for connection to said devices, which is separate from that used in the connection of the tubes (6) of the pressotherapy element.

The outer pressotherapy applicator element and inner cold and/or heat applicator element are joined together by a zip and/or velcro (9).

The novel garment is made in such a way that each of the two legs is vertically open, preferably along the outside thereof, so that it is easy for the user to put on; each of these vertical openings has three zips (14), so that the legs can be adjusted to the diameter of each user's leg.

The materials used to manufacture the different elements that it comprises are independent of the object of the present invention, as are the shape, dimensions and accessories that it might present, it being possible to replace them with other technically equivalent ones, provided that they do not affect the essence thereof and that they remain within the scope defined in the claims section.

Having established the aforementioned concept, the claims are stated below, thus summarising the novelties to be claimed:

## Claims

1. Garment for thermotherapy, cryotherapy and pressotherapy treatments, of the type used in health centres, spas and suchlike, essentially **characterised in that** it consists of the connection of two functional elements positioned concentrically one around the other, and both together around the user, the first element being a pressotherapy applicator (1) that is situated on the outside of the garment, and the other element being the thermotherapy and cryotherapy (2) applicator that is situated on the inside of the garment.

2. Garment for thermotherapy, cryotherapy and pressotherapy treatments according to the previous claim, **characterised in that** the pressotherapy applicator element is made from an impermeable fabric (3), such as plastic or suchlike, which is divided into a set of separate compartments or sealed chambers that are evenly distributed, exteriorly protected by a cover (4) to which the cryotherapy and thermotherapy applicator element (2) is coupled, attached to the inside of the pressotherapy element (1), and concentrically thereto, by a zip and/or velcro (9) or another means of fastening,

3. Garment for thermotherapy, cryotherapy and pressotherapy treatments according to the previous claims, **characterised in that** each chamber of the pressotherapy applicator element has a separate connector (5) to which a hose (6) can be connected to enable the independent Inflation thereof to apply the desired pressure, so that all the hoses (6) from the chambers are gathered and channelled inside a flap (7) provided on the front face of the cover (4).

4. Garment for thermotherapy, cryotherapy and pressotherapy treatments according to the previous claims, **characterised in that** the cryotherapy and thermotherapy applicator element (2) preferably consists of a coil (10) through which a thermostated fluid or gas flows, which is positioned between two covers: one inner (11) and one outer (12).

5. Garment for thermotherapy, cryotherapy and pressotherapy treatments according to the previous claims, **characterised in that** the inner cover (11) of the ' cryotherapy and thermotherapy applicator (2) is made from a material with optimum heat or cold transfer characteristics.

6. Garment for thermotherapy, cryotherapy and pressotherapy treatments according to the previous claims, **characterised in that** the coil (10) can alternatively be replaced or complemented by an electrical resistance.
